# EUROPEAN PATENT APPLICATION

(11) **EP 2 990 006 A1**
(43) Date of publication of application: **02.03.2016**
(21) Application number: 13882686.2
(22) Date of filing: 02.05.2013
(51) Int. Cl.: A61C 7/20, A61C 7/08, A61L 27/04

(54) **METHOD FOR MANUFACTURING COATING WIRE FOR CORRECTING TEETH ARRANGEMENT**

(30) Priority: 27.04.2013 KR 20130047067
(71) Applicant: Danybmt Co. Ltd., Anyang-si, Gyeonggi-do 431-810 (KR)
(72) Inventor: KIM, Tae Sik, Seoul 151-761 (KR); LEE, Yu Taek, Yongin-si Gyeonggi-do 446-010 (KR); NA, Jong Tak, Gunpo-si Gyeonggi-do 435-010 (KR)
(74) Representative: Petraz, Gilberto Luigi
(86) International application number: PCT/KR2013/003777
(87) International publication number: WO 2014/175494

(57) **Abstract**

The present invention relates to a method for manufacturing a coated orthodontic wire, and more particularly, to a method for manufacturing a coated orthodontic wire, in which the surface of a coated orthodontic wire, that is installed to connect teeth to each other to correct the alignment of the teeth, is etched, then plated with silver, and then coated with a polymer compound, so as to remarkably increase manufacturing speed and also allow the coating to be maintained for a long time due to the superior durability thereof. It may be expected from the above-mentioned configuration of the present invention and from the clinical results that there may be effects according to a method for manufacturing a coated orthodontic wire in that: a plurality of metal wires are fixed by using a jig, the metal wires are etched and plated in a state of being spaced apart from each other while only a required portion thereof is dipped into a container so that the manufacturing process is simple by virtue of not adding unnecessary processes, the manufacturing process is simple, only required portions of metal wires are etched, plated, and coated while the wires are fixed to be spaced apart from each other, and a low vacuum heat treatment step is performed one more time, thereby increasing the reliability of product quality. Since the polymer compound is completely coated through the low vacuum heat treatment step, the coated metal wire may be maintained for a long time even under a harsh oral environment.

## Description

### TECHNICAL FIELD

The present invention relates to a method for manufacturing a coated orthodontic wire, and more particularly, to a method for manufacturing a coated orthodontic wire, in which the surface of a coated orthodontic wire, that is installed to connect teeth to each other to correct the alignment of the teeth, is etched, then plated with silver, and then coated with a polymer compound, so as to remarkably increase manufacturing speed and also allow the coating to be maintained for a long time due to the superior durability thereof.

### BACKGROUND ART

Uneven arrangement of teeth or malocclusion are typically caused because the teeth do not grow in their correct positions due to dysplasia of the teeth, jaw dysplasia, bad habits in childhood such as finger sucking, bad eating habits, or other factors.

Having such an uneven arrangement of teeth or malocclusion may degrade one's social life due to passive reactions in interpersonal relationships caused by poor self-confidence or covering the mouth when talking to others or laughing. Also, when eating foods, the foods may not be uniformly crushed or food particles may become stuck between teeth to cause various dental diseases or disorders of the digestive system. The uneven arrangement of teeth or malocclusion upsets the overall balance of the body, causing one to suffer from various kinds of minor illnesses.

Accordingly, to address such issues, a correction technique is being used that is based on a principle of allowing the teeth to move with the reconstruction of the alveolar bone surrounding the teeth by continuously applying force to the teeth.

An orthodontic wire is used in the correction technique. A metal wire made of stainless steel or nickel-titanium (Ni-Ti) alloy is typically used for the orthodontic wire.

The orthodontic wire is formed to have a great torque transmission effect, great corrosion resistance even in poor oral environments, and a low amount of friction against a bracket fixed to the teeth so as to be appropriate for applying sliding mechanics.

Also, the orthodontic wires are sold in arch shapes having various sizes before being applied to the teeth, and dentists buy and use the orthodontic wires according to oral cavity size and application.

However, a recent trend is that patients prefer coated orthodontic wires which have non-metallic colors similar to those of teeth during a correction period.

The orthodontic wire is provided in such a way that the surface thereof is coated such that the outwardly visible portion looks similar to a nonmetallic tooth color while the wire retains the functional characteristics required for an orthodontic wire.

Typical technologies related to a coated orthodontic wire and a method for manufacturing the same have been disclosed, since around 1990, in U.S. Patent No. 5,063,082, U.S. Patent No. 4,946,387, and Korea Patent Nos. 0795106, 1033025, 0919900, 0666120, and 1136551.

Specifically, a method for manufacturing an orthodontic wire disclosed in Korea Patent No. 0795106 includes manufacturing a shape of a metal wire formed of stainless steel or nickel-titanium; coating the surface of the manufactured metal wire with a silver film; and forming a polymer compound film by coating a resin material on the surface of the silver film.

### DISCLOSURE OF THE INVENTION

### TECHNICAL PROBLEM

However, typical methods of manufacturing coated orthodontic wire and orthodontic wires manufactured by the same methods have the following limitations.

(1) Since production is done through a method in which a plurality of metal wires are dipped into an etching container or a plating container at a time, the manufacturing process is complicated, in which a polymer compound in a portion that does not require plating should be separately removed after a coating of the polymer compound is coated on the entirety of the metal wires.

(2) Manufacturing costs increase because the manufacturing process takes a long time due to the complexity thereof, and the yield and reliability with respect to product quality decrease because a polymer compound is not completely removed during the removal of a portion that does not require a coating of the polymer compound.

(3) A polymer compound is not properly coated, and thus a coating of the polymer compound does not last for a long time and is easily peeled off due to a harsh oral environment.

### TECHNICAL SOLUTION

An embodiment of the prevent invention provides a general method for manufacturing a coated orthodontic wire including: a metal wire manufacturing step of manufacturing a metal wire by using a shape-memory alloy; a plating step of coating a surface of the metal wire with a silver film; and a coating step of coating a surface of the silver film with a resin material to form a polymer compound film, wherein
an etching step of dipping the metal wire into an etching liquid is included after the metal wire manufacturing step, and a low vacuum heat treatment step of increasing the adhesiveness of the polymer compound is included after the coating step, wherein
the etching step includes: fixing a plurality of metal wires in a jig capable of supporting both end portions of the metal wire; and etching the metal wires while fixing the jig upside down such that a curved portion of the metal wire is dipped into an etching container filled with the etching liquid, wherein
the plating step includes plating the metal wires while fixing the jig upside down such that the etched curved portion of the metal wire is dipped into a plating container filled with a plating liquid in a state in which the metal wire is fixed, as-is, to the jig after etching, wherein
the coating step includes coating the silver-coated metal wires while vaporizing the polymer compound in a vacuum chamber.

### ADVANTAGEOUS EFFECTS

According to a method for manufacturing a coated orthodontic wire, the following effects may be attained.
(1) A plurality of metal wires are fixed by using a jig, the metal wires are etched and plated in a state spaced apart from each other while only a required portion thereof is dipped into a container, and thus the manufacturing process is simple by virtue of not adding unnecessary processes.
(2) The manufacturing process is simple, only the required portion of metal wires are etched, plated, and coated while fixed to be spaced apart from each other, and a low vacuum heat treatment step is performed one more time, thereby increasing the reliability of product quality.
(3) Since the polymer compound is completely coated through the low vacuum heat treatment step, the coated metal wire may be maintained for a long time even in a harsh oral environment.

### BRIEF DESCRIPTION OF THE DRAWINGS

FIG. 1 is a flow chart illustrating processes in a method for manufacturing a coated orthodontic wire formed according to an exemplary embodiment of the present invention.
FIG. 2 is a perspective view illustrating a coated orthodontic wire manufactured through a method for manufacturing a coated orthodontic wire formed according to an exemplary embodiment of the present invention.

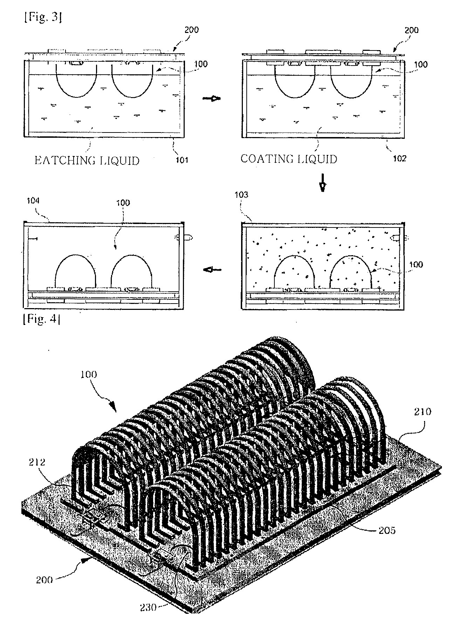

FIG. 3 is a process chart illustrating a method for manufacturing a coated orthodontic wire formed according to an exemplary embodiment of the present invention.
FIG. 4 is a perspective view illustrating a jig used in a method for manufacturing a coated orthodontic wire formed according to an exemplary embodiment of the present invention and a metal wire mounted to the jig.
FIG. 5 is a conceptual diagram illustrating a state in which a metal wire manufactured by a method for manufacturing a coated orthodontic wire formed according to an exemplary embodiment of the present invention is etched and plated.
FIG. 6 is a perspective view illustrating a retainer manufactured by a method for manufacturing a coated orthodontic wire formed according to an exemplary embodiment of the present invention.
FIG. 7 is a group of photographs illustrating a result of a clinical experiment on a coated orthodontic wire produced through a method for manufacturing a coated orthodontic wire according to an exemplary embodiment of the present invention.
FIG. 8 is a photograph illustrating another result of a clinical experiment of a coated orthodontic wire produced through a method for manufacturing a coated orthodontic wire formed according to an exemplary embodiment of the present invention.

### MODE FOR CARRYING OUT THE INVENTION

The present invention includes: a metal wire manufacturing step 10 of manufacturing a metal wire 100 by using a shape-memory alloy;
an etching step 20 of dipping a curved portion 110 of the metal wire 100 into an etching liquid and etching a dipped surface;
a plating step 30 of plating a surface of the curved portion 110 of the etched metal wire 100 with silver (Ag) ;
a coating step 40 of loading the silver-coated metal wire 100 into a vacuum chamber 103, and forming a polymer compound film by vaporizing the polymer compound; and
a low vacuum heat treatment step 50 of heat-treating in a low vacuum chamber 104 to increase the adhesiveness of the polymer compound.

In the metal wire manufacturing step 10, the metal wire 100 having a U-shape is manufactured by using stainless steel or a shape-memory alloy formed of nickel-titanium alloy or cobalt-chromium alloy.

The metal wire 100 is formed to have a rectangular or circular cross-sectional shape. The rectangular shape is preferred to increase adhesiveness, and the circular shape is preferred to increase rigidity.

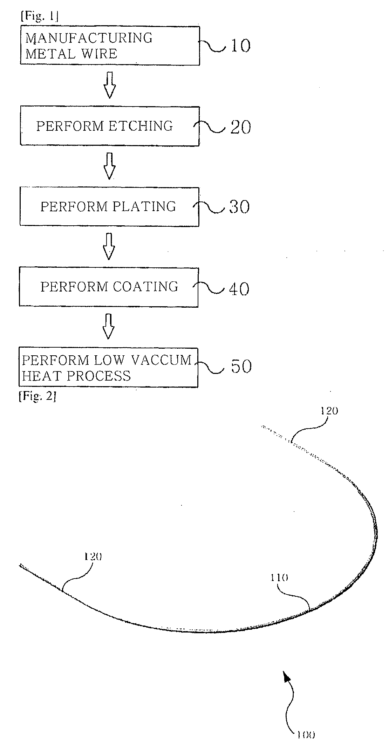

The metal wire 100 includes a curved portion 110 bent in a U-shape as illustrated in FIG. 2, and an extension portion 120 extending from both end portions of the curved portion 110.

Since the curved portion 110 is positioned at a front surface when fixed to the teeth and exposed to the outside, this portion is suitable for a coating in order that the color thereof is the same as the teeth.

The extension portion 120 of the metal wire 100 is used by being cut according to its purpose at a site for treatment.

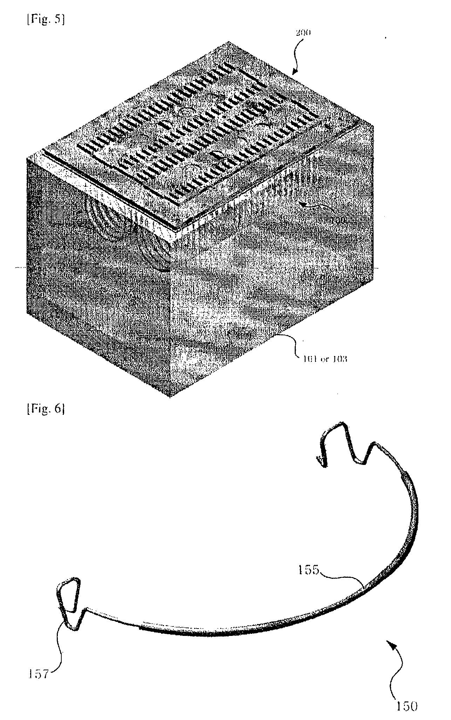

The metal wire may be also applied to a retainer 150 as illustrated in FIG. 6, and the retainer 150 includes a hanging portion 157 at an end of a curved portion 155 so as to be hung on the molar side of the teeth.

The etching step 30 is a preparation step for plating silver (Ag), and is a step of etching a surface of the curved portion 110.

When the metal wire is formed of stainless steel or a cobalt-chromium alloy, the etching liquid used for the etching step 30 is prepared, by mixing a nitric acid (NHO₃) and a hydrofluoric acid (HF) at a weight ratio of 5:1 and diluting the mixture of the nitric acid (NHO₃) and the hydrofluoric acid (HF) with water or an organic solvent, and When the metal wire is formed of a nickel-titanium alloy, the etching liquid is prepared by diluting a hydrofluoric acid (HF) with water or an organic solvent.

In the etching step 30, a plurality of metal wires 100 are fixed to a jig 200 capable of supporting an end of each extension portion 120 of the metal wire 100, and surfaces of the metal wires are then etched (or corroded) while the jig 200 is fixed upside-down such that portions of the curved portion 110 and extension portion 120 of the metal wire 100 are dipped into an etching container 101 filled with an etching liquid.

The jig 200 has a plurality of channels 205 in a longitudinal direction in a body panel 210, and a fixed panel 230 is provided below each channel 205.

Each of the channels 205 is fully opened, and a plurality of fixing gaps 212 are provided in a length direction along inner side surfaces thereof.

Each end of the extension portion 120 of the metal wire 100 is fixed to the corresponding fixing gaps 212, an end of the extension portion 120 of the metal wire 100 inserted into the fixing gap 212 is completely fixed by moving the fixing panel 230.

The fixing panel 230 is pushed toward each side surface to fix each end of the extension portion 120 inserted into the fixing gap 212, within the fixing gap. The fixing panel 230 may be provided as a locking means that fixes, from both sides of the channel 205 corresponding to the fixing gap 212, each end of the extension portion 120 inserted into the fixing gap 212.

The etching step 30 is performed for one to five minutes. When the etching is performed for too long, the surface is seriously damaged to lower product durability.

In the plating step 30 of plating a surface of the etched metal wire 100 with silver (Ag), a plating liquid, which is an aqueous solution of silver cyanide (Ag(CN)) prepared by melting a silver (Ag) rod into a potassium cyanide (KCN) solution in a plating container 102, is provided, and an electrode is applied for plating.

The plating step 30 is performed for four to six hours under room temperature, and a drying step of curing and drying for 24 hours is performed after the plating step 30.

When the plating step 30 is performed, the thickness of silver plated on surfaces of the metal wires 100 is about 2 µm to about 5 µm.

After undergoing the plating step 30, the curved portion 110 of the metal wire 100 adopts a color similar to the teeth.

The coating step 40 is a step of coating a polymer compound to improve the surface smoothness and the durability of the curved portion 110 and the extension portion 120 of the metal wire 100, and parylene is appropriate for use as the polymer compound.

Parylene has an advantage in that it is harmless to humans, has good surface roughness, and a good texture when worn.

Although various kinds of the parylene, such as C-type (Di-chloro-para-xylylene), N-type (Di-para-xylene), D-type (Tetra-chloro-para-xylylene), F-type (Octafluoro-[2,2]para-xylylene), may be used, the C-type is appropriate for use in the present invention.

In the coating step 40, parylene is vaporized at 100°C to 200°C in a vaporizer and is then allowed to pass through an electric furnace of 600°C, thereby being decomposed into a monomer. Then, the parylene is pushed in at a predetermined pressure through the metal wire 100 that has completed the plating step 30, and is deposited on the surface of the metal wire 100.

The coating step 40 lasts for five to 8 hours, immediately after which, the low vacuum heat treatment step 50 is performed.

The low vacuum heat treatment step 50 is a step of heat-treating the metal wire 100 coated with a parylene layer by loading the metal wire into a low vacuum chamber having a pressure lower than 0.1 mTorr and an internal temperature of 150°C to 200°C for two hours.

Hereinafter, the workings of a method for manufacturing a coated orthodontic wire according to an exemplary embodiment of the present invention and a performance test or the like for a coated orthodontic wire manufactured through such a manufacturing method will be described as follows.

The metal wire 100 is manufactured by using a shape-memory alloy, and the metal wire thus manufactured is loaded into a jig 200.

Here, each end of an extension part 120 of the metal wire 100 is loaded into each corresponding fixing gap 212 provided in a channel 205 of the jig 200, and all the fixing gaps 212 are loaded with the metal wires 100.

Then, fixing panels 230 are pulled to both sides to fix an end of the extension portion 120 of each metal wire 100 positioned in the fixing gaps 212.

In a state prepared as mentioned above, a jig 200 is placed upside-down on an upper end of an etching container 101 filled with an etching liquid, such that portions of the curved portion 110 and the extension portion 120 of the metal wire 100 are dipped into the etching liquid.

That is, the metal wire 100 is dipped in the etching liquid up to only the entire curved portion 110 and a portion of the extension portion 120, and the remaining extension portion 120 is not etched.

When the etching is thus completed, the jig 200 is loaded on an upper end of the plating container 102 containing the plating liquid in the same way it is loaded on the etching container 100, and plating is then performed.

Then, the plated metal wire 100 is loaded into a vacuum chamber 103, is then subjected to deposition and coated by injecting vaporized parylene, and is then loaded into a low vacuum chamber 104 to be stabilized and completed.

### [Clinical test result]

In order to compare the performance of a coated orthodontic wire manufactured by a manufacturing method according to the present invention and an orthodontic wire produced through a typical method, a coated orthodontic wire product (experimental group) according to the present invention and a typical orthodontic wire product (control group) were respectively implemented on the upper teeth and the lower teeth of a man in his 40s and a clinical test (Test 1) was performed at hospital "H".

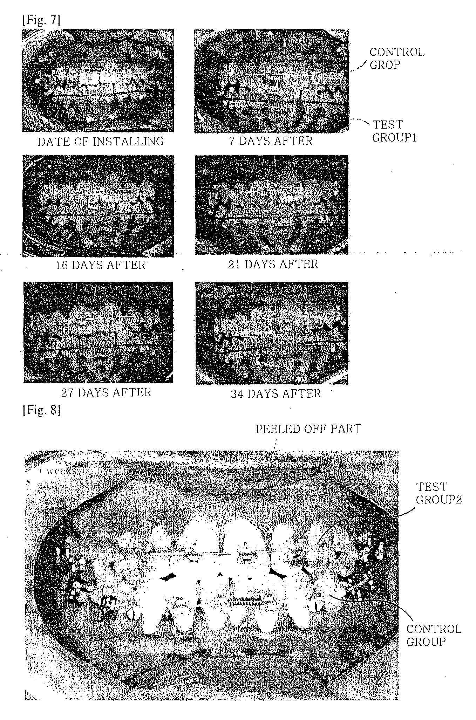

FIG. 7 illustrates a result of the clinical test after about five weeks from the start. It may be confirmed that the experimental group retained its surface color intact even under a harsh environment, and also fully functioned to correct the arrangement of teeth like the control group.

Also, in order to compare the coating retention performance of a coated orthodontic wire manufactured by a manufacturing method according to the present invention and a typical orthodontic wire, a typical orthodontic wire product (control group 2) produced through a typical method and the experimental group were respectively implemented on the upper teeth and the lower teeth of a man in his 30s and a clinical test (Test 2) was performed at hospital "S".

FIG. 8 is a photograph capturing results after performing the clinical test for four weeks. It may be confirmed that while the coating of a surface of the control group 2 is unsightly due to being peeled off in patches, the coating of the experimental group still remains and looks similar to the color of the teeth.

### [Summary of clinical test result]

As may be understood from the clinical test results, a coated orthodontic wire manufactured by a manufacturing method according to the present invention has no difference in terms of performance compared to a typical orthodontic wire (a wire showing a metallic color) as shown by the result of the test 1. Also, the coating of a coated orthodontic wire manufactured by a manufacturing method according to the present invention did not peel off at all even when the coating of a typical orthodontic wire (a wire showing a tooth color) manufactured through a typical manufacturing method was peeled off in test 2.

Accordingly, it may be proven that a coated orthodontic wire manufactured by a manufacturing method according to the present invention has that effect of having a coating that may be maintained for a long time while having sufficient rigidity.

It may be expected from the above-mentioned configuration of the present invention and from the clinical results that there may be effects according to a method for manufacturing a coated orthodontic wire in that: a plurality of metal wires are fixed by using a jig, the metal wires are etched and plated in a state of being spaced apart from each other while only a required portion thereof is dipped into a container so that the manufacturing process is simple by virtue of not adding unnecessary processes, the manufacturing process is simple, only required portions of metal wires are etched, plated, and coated while the wires are fixed to be spaced apart from each other, and a low vacuum heat treatment step is performed one more time, thereby increasing the reliability of product quality. Since the polymer compound is completely coated through the low vacuum heat treatment step, the coated metal wire may be maintained for a long time even under a harsh oral environment.

Although the present invention has been described with reference to the embodiments and the accompanying drawings, the present invention is not limited to these embodiments and the drawings. It should be understood that various modifications, additions and substitutions can be made by a person having ordinary knowledge in the art without departing from the scope and spirit of the invention as disclosed in the accompanying claims.

## Claims

1. A method for manufacturing a general orthodontic wire, the method comprising:
a metal wire manufacturing step of manufacturing a metal wire by using a shape-memory alloy;
a plating step of plating a surface of the metal wire with a silver film; and
a coating step of forming a polymer compound film through coating a surface of the silver film with a resin material, wherein
an etching step of dipping the metal wire into an etching liquid is provided after the metal wire manufacturing step, and
a low vacuum heat treatment step of increasing adhesiveness of the polymer compound is provided after the coating step, wherein
the etching step comprises fixing a plurality of metal wires in a jig capable of supporting both end portions of the metal wire; and etching the metal wires while fixing the jig upside-down such that curved portions of the metal wires are dipped into an etching container filled with the etching liquid, wherein
the plating step comprises plating the metal wires while fixing the jig upside down such that the etched curved portions of the etched metal wires are dipped into a plating container filled with a plating liquid in a state in which the metal wires are fixed, as-is, to the jig after etching, and wherein
the coating step comprises coating the silver-coated metal wires with a polymer compound by vaporizing the polymer compound in a vacuum chamber.

2. A method for manufacturing a general orthodontic wire, the method comprising:
a metal wire manufacturing step of manufacturing a metal wire by using a shape-memory alloy;
an etching step of dipping a curved portion of the metal wire in an etching liquid to etch only a surface portion dipped in the etching liquid;
a plating step of dipping only the portion of the metal wire etched in the etching step in a plating liquid to plate the portion with a silver film;
a coating step of loading the silver-plated metal wire in a vacuum chamber and vaporizing a polymer compound to form a polymer compound film on the silver-plated metal wire; and
a low vacuum heat treatment step of performing a heat treatment in a low vacuum chamber to increase adhesiveness of the polymer compound.

3. The method of claim 1, wherein
a jig used in the etching step and the plating step comprises:
a plurality of open channels in a longitudinal direction in a body panel;
at least one fixing gap provided symmetrically in a longitudinal direction along an inner side surface of each of the channels, and
a fixing means for fixing an end of the metal wire inserted into the fixing gap.

4. The method of claim 2, wherein
the fixing gap of the jig used in the etching step and the plating step is provided corresponding to each end of an extension portion of the metal wire to fix each end, and
the jig is provided to be loaded upside-down on upper ends of the etching container and the plating container.

5. The method of claim 1 or 2, wherein
when the metal wire is formed of stainless steel or a cobalt-chromium alloy, the etching liquid used in the etching step 30 is prepared by mixing a nitric acid (NHO₃) and a hydrofluoric acid (HF) at a weight ratio of 5:1 and diluting the mixture of the nitric acid (NHO₃) and the hydrofluoric acid (HF) with water or an organic solvent, and when the metal wire is formed of a nickel-titanium alloy, the etching liquid is prepared by diluting a hydrofluoric acid (HF) with water or an organic solvent.

6. The method of claim 1 or 2, wherein
the plating liquid used in the plating step is an aqueous solution of silver cyanide (Ag(CN)).

7. An orthodontic wire produced through the method of any one of claims 1 to 6.

8. An orthodontic retainer produced through the method of any one of claims 1 to 6.
